# EUROPEAN PATENT APPLICATION

(11) **EP 4 257 684 A1**
(43) Date of publication of application: **11.10.2023**
(21) Application number: 22166923.7
(22) Date of filing: 06.04.2022
(51) Int. Cl.: C12N 15/10

(54) **REAGENTS FOR SUBCELLULAR DELIVERY OF CARGO TO TARGET CELLS**

(71) Applicant: Eleven Therapeutics Ltd., Tel-Aviv-Yafo (IL)
(72) Inventor: Ziv, Omer, Cambridge (GB); Erlich, Yaniv, Ra'anana (IL); Alendar, Andrej, Cambridge (GB)
(74) Representative: Fabry, Bernd

(57) **Abstract**

The present invention relates to a method to selectively identify reagents from a candidate library capable of localizing to a desired subcellular compartment of a target cell comprising the following steps:
a) preparing and/or selecting target cells comprising a polymerase localizing to said desired compartment;
b) preparing a candidate library of reagents comprising or consisting of nucleic acid barcodes recognized by said polymerase;
c) contacting said target cells with said library of reagents, wherein at least a subset of reagents interacts with at least a subset of target cells forming cell-reagent complexes;
d) incubating the cell-reagent complexes thus obtained for a period of time at least sufficient to allow at least a subset of said reagents to enter a desired subcellular compartment of a target cell;
e) amplifying the nucleic acid barcode of said subset of reagents of step d) within the desired subcellular compartment of the target cells and
f) separating the amplified nucleic acid barcodes of step e) to generate and/or identify said reagent.
as well as reagents and applications of said reagents obtainable according to the disclosed method.

## Description

### FIELD OF THE INVENTION:

The present invention relates to a method for generating and/or identifying reagents able to penetrate a desired subcellular compartment of target cells for delivery of cargo *in vitro* and *in vivo.* The invention allows for the identification of successful candidate reagents such as small molecules or aptamers by amplifying and modifying the signal of a barcode attached to said reagents in the desired subcellular compartment thereby significantly increasing the signal to noise ratio and distinguishing the reagents that successfully entered the desired cells or the desired subcellular compartment. Disclosed herein are methods for generating and/or identifying a reagent, said reagent being able to enter a desired subcellular compartment of a target cell as well as therapeutic applications of said reagents.

### BACKGROUND OF THE INVENTION

Most therapeutics require delivery to target tissues or cells. In many cases the amounts of compounds necessary to achieve a therapeutic effect are relatively high since selective delivery is extremely difficult and often relies on targeting the whole body instead, which can result in many side effects. Additionally, biological structures targeted by therapeutics are often within cells. However, cell permeability of many compounds is limited due to biophysical properties. Therefore, there is an unmet demand by the pharmaceutical industry for the identification of reagents and delivery systems that are able to deliver cargo, such as therapeutically active effectors, to specific cells and subcellular compartments that can be produced efficiently, show high specificity to target cells or subcellular compartments, and low toxicity.

The use of DNA-encoded libraries (DEL) has improved the identification of molecules with certain functions. For this approach, a candidate reagent, typically a small molecule or a peptide, is conjugated with a unique barcode allowing the identification of said reagent through sequencing. This concept has been applied to high-throughput methods by generating candidate libraries comprising millions or billions of different reagents tagged by unique identifying barcode DNA or RNA sequences. Candidate libraries are then incubated with target proteins *in vitro.* Candidate reagents failing to bind are removed in a subsequent step followed by isolation of nucleic acid barcodes attached to the remaining reagents. Said recovered barcodes are then sequenced in order to identify reagents that successfully bound to the protein

However, while this approach represents a major improvement to conventional small molecule screening approaches, it is inadequate to identify cell-entering molecules due to several limitations: 1) removing unbound reagents is often insufficient to decrease all background signal. This background can be caused by reagents or barcodes that only bind the target cell but fail to internalize or non-specifically attach to the incubation/screening vessel such as plastic or glass dishes 2) Additionally, reagents that internalize through endocytosis but were not able to exit the endosome or reach the desired subcellular compartment of a target cell cannot be distinguished from reagents penetrating the desired compartment since separation between localization to different compartments using for instance mechanical approaches is not feasible with this method. Lastly, while DELs have been used extensively in high throughput screens *in vitro, in vivo* or *in cellulo* application is not feasible at the moment. Therefore, there is an unmet demand of methods able to identify reagents that successfully penetrate a desired subcellular compartment of target cells to, for instance, further deliver cargo molecules attached to said reagents such as therapeutically active effectors.

Reagents particularly suitable to deliver cargo to subcellular compartments of target cells are nucleic acids as their production is cost effective and the technology widely implemented. Additionally, the low immunological properties of nucleic acids limit adverse effects in clinical settings. Chemically modified nucleic acids can be easily attached to most cargo. Therefore, nucleic acids with the ability to bind and enter the cytoplasm or other compartments of a target cell are particularly interesting for therapeutic applications.

Aptamers are short oligonucleotides made of either RNA, DNA, Xeno Nucleic Acids (XNA), RNA/DNA/XNA chimeras, and/or chemically modified nucleic acids. Depending on the nucleic acid base sequence, these short molecules can fold into three dimensional structures and selectively interact with a target molecule such as a protein, lipid, or a small molecule. Certain aptamers bind to a cell membrane receptor and internalize into the cell via receptor mediated endocytosis. While aptamers by themselves can be potent therapeutics, previous studies have further utilized this capability and chemically attached therapeutic oligonucleotides to cell-internalizing aptamers as a means to deliver the payload in a cellspecific manner. However, thus far, the success of this strategy has been limited by the laborintensive aptamer discovery process *in vitro,* which requires multiple enrichment steps and has inherent specificity and selectivity limitations.

Historically, aptamer development has utilized Systematic Evolution of Ligands by Exponential Enrichment (SELEX) [Recent reviews: doi:10.1016/j.talanta.2019.03.015, doi:10.1016/j.biochi.2018.09.001]. This procedure starts with a candidate library of RNA or DNA oligos bearing a random 20-60 nucleotide long region. Such libraries typically contain about 10 to the power of 15 oligos, which is orders of magnitude smaller than all possible sequence combinations. Consequently, the vast majority of sequences are represented by a single molecule each within the library. The aptamer library is then reacted with a pre-selected receptor such as an immobilized protein or cell type, after which, unbound aptamers are washed away, while bound candidates are eluted and amplified by polymerase chain reaction (PCR). Amplified aptamers are then purified and sequenced to identify the unique sequence of each successful candidate aptamer.

In order to enrich for aptamers with the desired properties, the process above is repeated multiple times by utilizing the successful aptamer candidates form the previous cycle until convergence is achieved.

While this method has been powerful to identify aptamers with high affinity for their selected targets such as proteins, the SELEX platform suffers from sensitivity-selectivity tradeoffs similar to those discussed above for DELs: Aptamers that bind non-specifically to the screening vessel or the target cells but fail to internalize, significantly increase the background over the signal of successful candidate aptamers. The sheer number of initial molecules requires multiple selection rounds to eliminate the bulk of non-specific aptamers. Each round involves many PCR amplification cycles, which induce a strong genetic bottleneck and can eliminate highly potent aptamers. As such, the sensitivity of the screen diminishes, resulting in suboptimal selection of aptamers from the initial starting population of aptamers. Moreover, the SELEX approach does not directly select for aptamers with ability to penetrate cells and release their payload into the desired subcellular compartments such as the cytoplasm. However, such functional properties are essential for selective drug delivery.

Further variations of the SELEX basic method were successful to address some of these concerns. For instance, the development of cell-internalization SELEX allows screening for aptamer populations, that are more likely to be internalized by target cells thereby identifying more promising candidates for cargo delivery (Thiel et al., 2012). Only a small subset of candidate aptamers binds target structures and each sequence is present as a single molecule making identification of true positive candidate aptamers highly dependent on contaminating background from non-specifically bound aptamers. Cell-internalization SELEX can reduce some background, which is achieved by stringent washing procedures following aptamer incubation with target cells, which strips bound aptamers from the cell surface while not affecting internalized candidates. Aptamers identified by this method were more likely to internalize into cells and showed some ability to deliver siRNA as cargo to cancer cells. While cell internalization is required for cargo delivery to target cells, the escape of the aptamer or aptamer-cargo chimera to the cytoplasm is essential to fulfill almost all therapeutic functions within the cell. However, cell-internalization SELEX does not screen for aptamers that exit the endosome and suffers similar genetic bottlenecks as described above, which is specifically highlighted by the authors and further indicates a high demand for more suitable aptamers able to not only internalize but also escape the endosome, which has not been successfully addressed within the decade since the publication was released. Lastly, said SELEX method again suffers from sensitivity issues since aptamers that successfully entered the cell are present typically as a single copy and identifying successful candidates is highly challenging, if at all possible, even with the improvement of background reduction. Therefore, amplifying extremely low signal over the background from undesired non-specifically binding aptamers is not feasible with the methods available to this date.

Therefore, while SELEX is a preferred and well-established method to identify potential aptamer candidates *in vitro* with binding affinity for selected targets, the current protocols for this method are unable to address either the lengthy and costly process associated with this method, limitation on *in vitro* screens only, or the failure to identify aptamers that escape form the endosome, essential to unlock the full therapeutic potential of aptamers.

The underlying objective of the present invention was therefore, to develop a method allowing to improve identification approaches for reagents such as aptamers, small molecules, and peptides able to enter a desired subcellular compartment of a target cell applicable to *in vitro* and *in vivo* screens, thereby improving the sensitivity of detection methods such as commonly used in DEL- or SELEX-based approaches.

### DEFINITIONS

For convenience, certain terms employed in the specification, examples, and appended claims are collected here.

As used herein, the singular form "a," "an," and "the" include plural references unless the context clearly dictates otherwise. For example, the term "target cell" also includes a plurality of target cells.

The terms "polynucleotide" and "nucleic acid" are used herein interchangeably. They refer to a polymeric form of nucleotides of any length: Polynucleotides may have any three-dimensional structure, and may perform any function, known or unknown. The following are non-limiting examples of polynucleotides: coding or non-coding regions of a gene or gene fragment, exons, introns, messenger RNA (mRNA), transfer RNA, ribosomal RNA, ribozymes, cDNA, synthetic polynucleotides, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified, such as by conjugation with a labeling component.

If not stated otherwise, the term "nucleic acid" refers to any nucleic acid such as ribonucleic acid, deoxyribonucleic acid, xeno nucleic acid, single stranded or double stranded.

As used herein, the term "xeno nucleic acids" or "XNAs" are synthetic nucleic acid analogues that have a different phospho-sugar backbone or nucleobases than the natural nucleic acids DNA and RNA.

As used herein, two nucleic acid sequences "complement" one another or are "complementary" to one another if they base pair one another at each position. The term "complement" is defined as a sequence which pairs to a given sequence based upon the canonic base-pairing rules. For example, a sequence A-G-T in one nucleotide strand is "complementary" to T-C-A in the other strand The term "complement" and the phrase "reverse complement" are used interchangeably herein with respect to nucleic acids, and are meant to define the antisense nucleic acid.

As used herein, the term "aptamer" refers to a single stranded and/or double stranded nucleic acid molecule (e.g., ssDNA, ssRNA and/or chimeras of ssRNA and dsDNA) able to specifically bind a structure such as proteins, peptides, nucleic acids, lipids and/or topographic features on a target cell.

The concept of bivalent or multivalent aptamers was proposed and verified in a patent in 1999 by Shi et al., which are defined as modified aptamer structures consisting of two or more identical or different single aptamers, with or without additional structures

As used herein, the term "desired reagent" or " reagent" refers to a reagent identified or generated according to the method of the present invention, characterized in that it localizes to a desired subcellular compartment of a target cell.

As used herein, the term "candidate reagent library" or" reagent library" or" candidate library" refers to a mixture of reagents or compounds such as, but not limiting, small molecules, proteins, peptides, lipids, polymers or nucleic acids of differing sequence or mixtures thereof, from which to select a desired reagent. A library comprises at least one reagent. Non-limiting examples for candidate libraries are aptamer libraries or DNA-encoded libraries (DELs).

As used herein, the term "barcode" refers to a nucleic acids sequence used to identify reagents. Barcodes can be chemically attached to reagents or form electrostatic interactions with the reagent, or can be encapsulated by the reagent. Each reagent can be identified by the barcode sequence attached. Reagents can be barcodes by themselves for instance if the reagent comprises a nucleic acid sequence such as aptamers.

As used herein, the term "DNA-encoded library" or "DEL" refers to an embodiment of a candidate reagent library. A DNA-encoded library can comprise a collection of small molecules that are conjugated covalently to DNA tags that serve as identification barcodes.

As used herein, the term "aptamer library" refers to an embodiment of a candidate reagent library. An aptamer library can comprise a collection of nucleic acid molecules, wherein sequences can be different for each molecule. Libraries can have a size as measured in number of molecules from at least one aptamer. The aptamers synthesized in an aptamer library may contain any domain which has a biological function. Non-limiting examples of biological functions of the aptamers described herein include, but are not limited to, localizing to a subcellular compartment, binding to a cell, inducing endocytosis, escaping from the endosome, acting as templates for RNA transcription, binding to, recognizing, and/or modulating the activity of proteins, binding to transcription factors, specialized nucleic acid structure (e.g., Z-DNA, H-DNA, G-quad, etc.), acting as an enzymatic substrate for restriction enzymes, specific exo- and endonucleases, recombination sites, editing sites, or siRNA.

As used herein, the term "contacting" refers to bringing together two or more molecular entities such that they can interact with each other. Non-limiting examples for molecular entities are reagents according to the preset invention such as aptamers, proteins, nucleic acids, lipids and cells.

The term "binding" or "interacting" refers to an association, which may be an association, between two molecules, e.g., between an aptamer and target, due to, for example, electrostatic, hydrophobic, ionic and/or hydrogen-bond interactions under physiological conditions.

As used herein, the term "click chemistry" refers to bio-orthogonal reactions known to those of ordinary skill in the art.

As used herein, the term "amplifying" refers to any process or combination of process steps that increases the amount or number of copies of a molecule such as a nucleic acid. Polymerase chain reaction (PCR) is an exemplary method for amplifying of nucleic acids.

As used herein, the term "in-cell amplification" or "in-cell amplifying" refers to the process of making copies of barcodes, wherein the generation of at least one copy of the target barcode is considered as amplified. The copy can be a reverse complement version of the barcode, and can be composed of a different nucleic acid (for example the original barcode is DNA and the copy is made of RNA).

As used herein, the term "separating" refers to any process whereby specific subsets of molecules such as nucleic acids, e.g., barcodes attached to reagents localizing to the desired subcellular compartment of a target cell or amplified copies of said barcodes, can be separated from other subsets of molecules. Separating for example can be accomplished by selectively immobilization of the nucleic acid subset on magnetic beads or by capillary electrophoresis of the desired nucleic acids.

As used herein, the term "cell reagent complex" refers to an interaction between the reagent and the cell including binding to a structure of the cell surface or transient interactions such as active or passive diffusion through the cell membrane.

As used herein, the term "cargo" refers to any molecule considered for cellular delivery that can be functionally attached to a reagent according to the present invention. Non-limiting examples for possible cargo are nucleic acids, proteins, lipids and small molecules.

As used herein, the term "desired (sub)cellular compartment" or "desired compartment" refers to any subcellular compartment of a cell for which a reagent according to the present invention is sought and for which targeted localization of proteins such as a polymerase is achievable through methods known in the art. A desired subcellular compartment can also refer to multiple compartments. Non-limiting examples of desired subcellular compartments are cytoplasm, nucleus, Golgi apparatus, endoplasmic reticulum, mitochondria, chloroplast, vacuole, membrane microdomains, nucleolus

As used herein, a functionally enriched population refers to a mixture of reagents such as small molecules, aptamers or the barcode attached to the reagents, which is enriched for localizing to a desired subcellular compartment according to the present invention.

As used herein "therapeutically effective amount" refers to an amount of a composition that relieves (to some extent, as judged by a skilled medical practitioner) one or more symptoms of the disease or condition in a mammal. Additionally, by "therapeutically effective amount" of a composition is meant an amount that returns to normal, either partially or completely, physiological or biochemical parameters associated with or causative of a disease or condition. A clinician skilled in the art can determine the therapeutically effective amount of a composition in order to treat or prevent a particular disease condition, or disorder when it is administered, such as intravenously, subcutaneously, intraperitoneally, orally, or through inhalation. The precise amount of the composition required to be therapeutically effective will depend upon numerous factors, e.g., such as the specific activity of the active agent, the delivery device employed, physical characteristics of the agent, purpose for the administration, in addition to many patient-specific considerations. But a determination of a therapeutically effective amount is within the skill of an ordinarily skilled clinician upon the appreciation of the disclosure set forth herein.

The terms "treating," "treatment," "therapy," and "therapeutic treatment" as used herein refer to curative therapy, prophylactic therapy, or preventative therapy. An example of "preventative therapy" is the prevention or lessening the chance of a targeted disease (e.g., cancer or other proliferative disease) or related condition thereto. Those in need of treatment include those already with the disease or condition as well as those prone to have the disease or condition to be prevented. The terms "treating," "treatment," "therapy," and "therapeutic treatment" as used herein also describe the management and care of a mammal for the purpose of combating a disease, or related condition, and includes the administration of a composition to alleviate the symptoms, side effects, or other complications of the disease, condition. Therapeutic treatment for cancer includes, but is not limited to, surgery, chemotherapy, radiation therapy, gene therapy, and immunotherapy.

The term "amplification initialization element" or "regulatory elements" describes any nucleic acid sequence useful for the recognition of a polymerase according to the present invention in order to initiate amplification.

Unless defined otherwise, the technical and scientific terms used herein have the same meaning as commonly understood by one of ordinary skill in the art to which the invention belongs. Particularly, unless otherwise stated, a term as used herein is given the definition as provided in the Oxford dictionary of biochemistry and molecular biology, Oxford University Press, 1997, revised 2000 and reprinted 2003, ISBN 0 19 850673 2.

### DETAILED DESCRIPTION OF THE INVENTION

A first object of the present invention refers to a method for generating and/or identifying a reagent, said reagent being able to enter a desired subcellular compartment of a target cell, comprising the following steps:
a) preparing and/or selecting target cells comprising a polymerase localizing to said desired compartment;
b) preparing a candidate library of reagents comprising or consisting of nucleic acid barcodes recognized by said polymerase;
c) contacting said target cells with said library of reagents, wherein at least a subset of reagents interacts with at least a subset of target cells forming cell-reagent complexes;
d) incubating the cell-reagent complexes thus obtained for a period of time at least sufficient to allow at least a subset of said reagents to enter a desired subcellular compartment of a target cell;
e) amplifying the nucleic acid barcode of said subset of reagents of step d) by the said polymerase of step a) within the desired subcellular compartment of the target cells and
f) separating the amplified nucleic acid barcodes of step e) to generate and/or identify said reagent.

The method according to the present invention relies on the specific and autonomous amplification of nucleic acid barcodes attached to the reagents of a candidate library entering the desired subcellular compartments within the target cells during the selection screen. Each barcode sequence can be attributed to a specific reagent, thereby making each said reagent identifiable by sequence. Candidates that reach a desired subcellular compartment are selectively amplified in said desired compartment of a target cell by a specific polymerase localizing to said compartment and recognizing regulatory elements comprising said barcodes. The in-cell autonomous amplification step highly specifically increases the copy number of barcodes of reagent candidate molecules that entered the desired subcellular compartment, while barcodes of reagents that fail to penetrate said compartments are not amplified. Amplified barcode copies can be separated and detected using sequencing methods known in the art. Amplification of successful candidate barcodes significantly increases the sensitivity of detection over non-specific reagent candidates that were not able to make contact with the polymerase.

Amplification of barcodes that localize to the desired subcellular compartment within a target cell (in-cell amplification) is achieved by providing the barcode with a customizable sequence, which can be recognized by a selected polymerase localizing to said desired compartment. Thereby, the present invention not only increases sensitivity of the method but also addresses previous limitation present in the art: The specific amplification exclusively in desired compartments reduces false-positive identification of reagents that for instance simply bind a target cell, bind non-specifically to the screening vessel, failed to internalize and/or did not escape the endosome, thereby allowing the identification of reagents able to guide cargo to any accessible cellular compartment.

It has been surprisingly found that the method according to the present invention is able to significantly increase detection sensitivity necessary to generate and/or identify reagents from a starting candidate library of reagents during a screening procedure that are able to enter a desired subcellular compartment of cells (target cells) incubated with said candidate reagents. State-of-the-art methods previously suffered from low sensitivity towards the detection of successful reagent candidates. Therefore, it is especially surprising that the method according to the present invention is able to identifying successful candidates in a high throughput-compatible manner by highly specifically increasing the copy number of barcodes corresponding to successful candidate reagents in desired subcellular compartments of target cells.

Some nucleic acids such as aptamers have intrinsic properties allowing delivery of cargo due to their three-dimensional shape. However, identifying sequences able to penetrate specific cellular compartments of target cells requires screening a massive number of different sequences and high sensitivity for the identification of the few expected candidates providing such properties.

It has been surprisingly found that the method according to the present invention is able to generate and identify aptamer populations from a starting candidate library of aptamers that show a high degree of endosomal escape to the cytoplasm or other compartments of cells incubated with said candidates (target cells). This is especially surprising since state-of-the-art SELEX approaches or other methods known in the art were previously unable to screen aptamer libraries for the ability to escape from the endosome due to sensitivity restrictions as well as failure to resolve spatial resolutions such as individual cellular compartments.

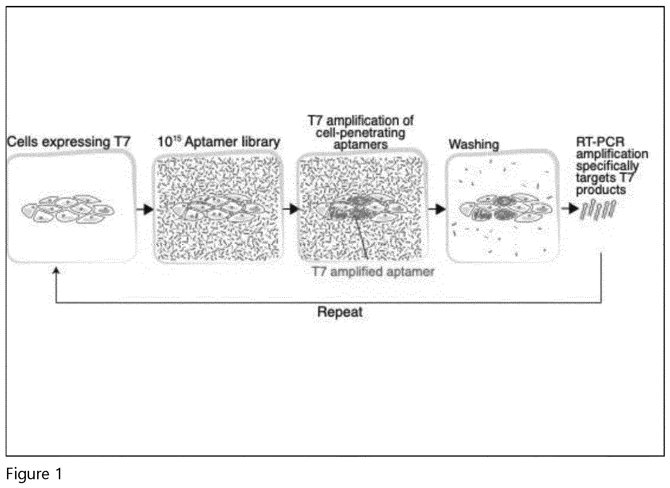

In some embodiments, the method according to the present invention enables the specific amplification of cell-penetrating and endosome-escaping aptamers sequences by the target cells themselves during the aptamer selection screen. The in-cell amplification step e) according to the present invention highly specifically increases the copy number of aptamer candidate molecules that escaped from the endosome and localized to desired compartments expressing the polymerase, while aptamers that cannot bind target cells, fail to internalize, cannot escape the endosome and/or localize to undesired compartments are not amplified. In addition, the polymerase generates a reverse complement template (amplicon) that contains unique and known sequences that are not in the original aptamer library, thereby allowing for their selective amplification. This allows increasing the signal of aptamers with desired properties over undesired aptamer sequences present in the library (see also **Figure 1****).**

It has been surprisingly found that the combination of (i) in-cell amplification and (ii) selective readout of the in-cell amplification products (amplicons) promises a superior sensitivity-selectivity balance than traditional SELEX. The present invention is designed to select only reagents such as aptamers capable of both cell entry and endosomal escape (to enable in-cell amplification during the screen), two crucial properties of nucleic acid delivery aptamers. Furthermore, due to the strong in-cell amplification, endosomal escape aptamers can be detected for weeks after their introduction into cells. Such prolonged duration can facilitate the selection of aptamers with high stability suitable for slow drug release.

State-of-the-art SELEX approaches require multiple cycles of purification of successful candidate aptamers and re-screening said candidates again to enrich for aptamers binding a desired target partially due to a high number of false-positive candidates associated with the high background and low sensitivity of the method. These cycles make SELEX a lengthy, costintensive and complicated approach for the identification of aptamers. Therefore, it is especially surprising that the method according to the present invention significantly reduces the number of cycles necessary to identify highly specific aptamer candidates, thereby decreasing cost, labor and increasing specificity of the functionally enriched aptamer population for endosomal escape and localization to desired compartments in target cells.

The method according to the present invention comprises several steps. In a first step a), a target cell comprising a polymerase localizing to a desired compartment for which a reagent that penetrates said desired subcellular compartment is sought, is selected and/or prepared.

In some embodiments a "target cell," as used herein, denotes an *in vivo* or *in vitro* eukaryotic cell, a prokaryotic cell (e.g., bacterial or archaeal cell), cells that comprise an organ on a chip, or a cell from a multicellular organism cultured as a unicellular entity (e.g., a cell line), which eukaryotic or prokaryotic cells can be, or have been used as recipients for a nucleic acid, and include the progeny of the original cell which has been transformed by the nucleic acid. It is understood that the progeny of a single cell may not necessarily be completely identical in morphology or in genomic or total DNA complement as the original parent, due to natural, accidental, or deliberate mutation.

In some embodiments, the target cell is a prokaryotic cell. In some embodiments, the cell is a bacterial cell. Non-limiting examples of bacteria include Aspergillus, Brugia, Candida, Chlamydia, Coccidia, Cryptococcus, Dirofilaria, Gonococcus, Histoplasma, Klebsiella, Legionella, Leishmania, Meningococci, Mycobacterium, Mycoplasma, Paramecium, Pertussis, Plasmodium, Pneumococcus, Pneumocystis, Pseudomonas, Rickettsia, Salmonella, Shigella, Staphylococcus, Streptococcus, Toxoplasma and Vibriocholerae. Exemplary species include Neisseria gonorrhea, Mycobacterium tuberculosis, Candida albicans, Candida tropicalis, Trichomonas vaginalis, Haemophilus vaginalis, Group B Streptococcus sp., Microplasma hominis, Hemophilus ducreyi, Granuloma inguinale, Lymphopathia venereum, Treponema pallidum, Brucella abortus. Brucella melitensis, Brucella suis, Brucella canis, Campylobacter fetus, Campylobacter fetus intestinalis, Leptospira pomona, Listeria monocytogenes, Brucella ovis, Chlamydia psittaci, Trichomonas foetus, Toxoplasma gondii, Escherichia coli,Actinobacillus equuli, Salmonella abortus ovis, Salmonella abortus equi, Pseudomonas aeruginosa, Corynebacterium equi, Corynebacterium pyogenes, Actinobaccilus seminis, Mycoplasma bovigenitalium, Aspergillus fumigatus, Absidia ramosa, Trypanosoma equiperdum, Babesia caballi, Clostridium tetani, and Clostridium botulinum. In some embodiments, the cell is a eukaryotic cell. In some embodiments, the cell is an animal cell ( e.g ., a mammalian cell). In some embodiments, the cell is a human cell. In some embodiments, the cell is from a nonhuman animal, such as a mouse, rat, rabbit, pig, bovine (e.g., cow, bull, buffalo), deer, sheep, goat, llama, chicken, cat, dog, ferret, or primate (e.g., marmoset, rhesus monkey). In some embodiments, the cell is a parasite cell (e.g., a malaria cell, a leishmanias cell, a Cryptosporidium cell or an amoeba cell). In some embodiments, the cell is a fungal cell, such as, e.g., Paracoccidioides brasiliensis.

In some embodiments, the target cell is a cancer cell (e.g., a human cancer cell or a patient- derived cancer cell). In some embodiments, the cell is from any cancerous or precancerous tumor. Non-limiting examples of cancer cells include cancer cells from the bladder, blood, bone, bone marrow, brain, breast, colon, esophagus, gastrointestine, gum, head, kidney, liver, lymph nodes, lung, nasopharynx, neck, ovary, pancreas, prostate, skin, stomach, testis, tongue, or uterus. In addition, the cancer may specifically be of the following histological type, though it is not limited to these: neoplasm, malignant, carcinoma, carcinoma, undifferentiated, giant and spindle cell carcinoma, small cell carcinoma, papillary carcinoma, squamous cell carcinoma, lymphoepithelial carcinoma, basal cell carcinoma, pilomatrix carcinoma, transitional cell carcinoma, papillary transitional cell carcinoma, adenocarcinoma, gastrinoma, malignant, cholangiocarcinoma, hepatocellular carcinoma, combined hepatocellular carcinoma and cholangiocarcinoma, trabecular adenocarcinoma, adenoid cystic carcinoma, adenocarcinoma in adenomatous polyp, adenocarcinoma, familial polyposis coli, solid carcinoma, carcinoid tumor, malignant, branchiolo-alveolar adenocarcinoma, papillary adenocarcinoma, chromophobe carcinoma, acidophil carcinoma, oxyphilic adenocarcinoma, basophil carcinoma, clear cell adenocarcinoma, granular cell carcinoma, follicular adenocarcinoma, papillary and follicular adenocarcinoma, nonencapsulating sclerosing carcinoma, adrenal cortical carcinoma, endometroid carcinoma, skin appendage carcinoma, apocrine adenocarcinoma, sebaceous adenocarcinoma, ceruminous adenocarcinoma, mucoepidermoid carcinoma, cystadenocarcinoma, papillary cystadenocarcinoma, papillary serous cystadenocarcinoma, mucinous cystadenocarcinoma, mucinous adenocarcinoma, signet ring cell carcinoma, infiltrating duct carcinoma, medullary carcinoma, lobular carcinoma, inflammatory carcinoma, paget's disease, mammary, acinar cell carcinoma, adenosquamous carcinoma, adenocarcinoma w/squamous metaplasia, thymoma, malignant, ovarian stromal tumor, malignant, thecoma, malignant, granulosa cell tumor, malignant, and roblastoma, malignant, sertoli cell carcinoma, leydig cell tumor, malignant, lipid cell tumor, malignant, paraganglioma, malignant, extramammary paraganglioma, malignant, pheochromocytoma, glomangiosarcoma, malignant melanoma, amelanotic melanoma, superficial spreading melanoma, malig melanoma in giant pigmented nevus, epithelioid cell melanoma, blue nevus, malignant, sarcoma, fibrosarcoma, fibrous histiocytoma, malignant, myxosarcoma, liposarcoma, leiomyosarcoma, rhabdomyosarcoma, embryonal rhabdomyosarcoma, alveolar rhabdomyosarcoma, stromal sarcoma, mixed tumor, malignant, mullerian mixed tumor, nephroblastoma, hepatoblastoma, carcinosarcoma, mesenchymoma, malignant, brenner tumor, malignant, phyllodes tumor, malignant, synovial sarcoma, mesothelioma, malignant, dysgerminoma, embryonal carcinoma, teratoma, malignant, struma ovarii, malignant, choriocarcinoma, mesonephroma, malignant, hemangiosarcoma, hemangioendothelioma, malignant, kaposi's sarcoma, hemangiopericytoma, malignant, lymphangiosarcoma, osteosarcoma, juxtacortical osteosarcoma, chondrosarcoma, chondroblastoma, malignant, mesenchymal chondrosarcoma, giant cell tumor of bone, ewing's sarcoma, odontogenic tumor, malignant, ameloblastic odontosarcoma, ameloblastoma, malignant, ameloblastic fibrosarcoma, pinealoma, malignant, chordoma, glioma, malignant, ependymoma, astrocytoma, protoplasmic astrocytoma, fibrillary astrocytoma, astroblastoma, glioblastoma, oligodendroglioma, oligodendroblastoma, primitive neuroectodermal, cerebellar sarcoma, soft tissue sarcoma, ganglioneuroblastoma, neuroblastoma, retinoblastoma, olfactory neurogenic tumor, meningioma, malignant, neurofibrosarcoma, neurilemmoma, malignant, granular cell tumor, malignant, malignant lymphoma, Hodgkin's disease, Hodgkin's lymphoma, paragranuloma, malignant lymphoma, small lymphocytic, malignant lymphoma, large cell, diffuse, malignant lymphoma, follicular, mycosis fungoides, other specified non-Hodgkin's lymphomas, malignant histiocytosis, multiple myeloma, mast cell sarcoma, immunoproliferative small intestinal disease, leukemia, lymphoid leukemia, plasma cell leukemia, erythroleukemia, lymphosarcoma cell leukemia, myeloid leukemia, basophilic leukemia, eosinophilic leukemia, monocytic leukemia, mast cell leukemia, megakaryoblastic leukemia, myeloid sarcoma, and hairy cell leukemia.

In some embodiments, the target cell is an immune cell (e.g., a human immune cell or a patient-derived immune cell). As used herein, the term "immune cell" refers to cells that play a role in the immune response. Immune cells are of hematopoietic origin, and include lymphocytes, such as B cells and T cells; natural killer cells; myeloid cells, such as monocytes, macrophages, eosinophils, mast cells, basophils, and granulocytes.

In some embodiments, the target cell is susceptible for viral infection or is infected by a virus. For example, in some embodiments, the virus is SARS-CoV-2, SARS-CoV-1, HIV, hepatitis A, hepatitis B, hepatitis C, herpes virus (e.g., HSV-1, HSV-2, CMV, HAV-6, VZV, Epstein Barr virus), adenovirus, influenza virus, flavivirus, echovirus, rhinovirus, coxsackie virus, coronavirus, respiratory syncytial virus, mumps virus, rotavirus, measles virus, rubella virus, parvovirus, vaccinia virus, HTLV, dengue virus, papillomavirus, molluscum virus, poliovirus, rabies virus, JC virus, or Ebola virus.

In some embodiments a target cell denotes multiple cells and tissues of an organism or all cells of an organism such as an animal or human. In some embodiments the target cell is a tissue comprising multiple cell types or an artificial tissue such as organoids. In some embodiment the target cell is cultured in a screening vessel such a plastic or glass dish. In some embodiments the target cell is growing within the natural or artificially altered environment of the tissue and/or organism.

In a preferred embodiment the target cell is a eukaryotic cell, preferably a cell of mammalian origin.

In some embodiments the target cells used is selected or prepared to express a polymerase.

In some embodiments said polymerase is of cellular or foreign origin.

In some embodiments genetic sequences encoding said polymerase can be permanently introduced into the genome of said target cell using genome engineering approaches such as TALEN, Zinc finger nucleases or CRISPR/Cas-related methods or by retroviral infection, such as lentivirus.

In a further embodiment polymerase can be delivered to the target cell as a protein or nucleic acids, such as cDNA or mRNA, coding for the respective polymerase.

In some embodiments the polymerase is a nucleic acid dependent nucleic acid polymerase. In some embodiments the polymerase is selected from the list of DNA dependent DNA polymerase, DNA dependent RNA polymerase, RNA dependent DNA polymerase or RNA dependent RNA polymerase. In some embodiments the polymerase is a replication complex made of more than one subunit (e.g., viral replication machinery).

In preferred embodiments the polymerase is selected from the list of T7 RNA polymerase, Phi29 DNA polymerase, Syn5, or viral replicases such as alphavirus replicase.

It has been surprisingly found that certain polymerases, in particular T7 RNAP, with RNA-dependent RNA activity can be utilized for in-cell amplification in order to generate reverse complement copies of barcodes that contain an accessible priming sequence for reverse transcription, which is inaccessible in the original barcode sequence (in sense direction), thereby allowing the exclusive separation of barcodes amplified by said polymerase by reverse transcription and subsequent identification of said barcode sequences. Therefore, in an even more preferred embodiment the polymerase according to the present invention is a T7 RNA polymerase (T7 RNAP). In some embodiments, said T7 polymerase can be mutated to modulate its activity, such as, but not limited to, the Ser43Tyr mutation in T7 RNAP.

Expression of said polymerase in a certain desired subcellular compartment (such as the nucleus, the cytosol, etc.) of the target cell can facilitate the selection of reagents capable of entering said subcellular compartment. A desired subcellular compartment (or desired compartment) is any subcellular structure enclosed by the cell membrane of the target cell for which a penetrating reagent is sought.

In-cell amplification of reagent barcodes is dependent on contact between the barcode and said polymerase as described above. Changes in the localization of said polymerase, therefore, can be used to selectively amplify reagent barcode subsets localizing to preferred subcellular compartments. Localization of said polymerase can be directed to individual compartments by common methods known in the art such as localization tags (e.g., Nuclear Localization Sequence (NLS), Nuclear Export Sequence (NES)).

In a preferred embodiment of the present invention proteins of said polymerase are ubiquitously abundant within all compartments of the target cell, in a more preferred embodiment said polymerase proteins are present in at least one cellular compartment or combinations thereof. In a more preferred embodiment said polymerase proteins are present in at least one of the compartments selected from the list of cytoplasm, nucleus, Golgi apparatus, endoplasmic reticulum, mitochondria or combinations thereof.

In a further step b) a suitable reagent library is generated. Said library comprising at least one reagent attached to at least one nucleic acid barcode able to identify said reagents by the barcode sequence(s). In some embodiments library size as measured in number of molecules is at least 10 to the power of 1 reagents, more preferred at least 10 to the power 5 reagents.

The barcode according to the present invention comprises a unique nucleic acid sequence able to identify attached reagents and optionally at least one amplification initialization element recognized by said polymerase. In a preferred embodiment said element is an origin of replication and/or a promoter transcription initiation site such as a T7 promoter sequence. In some embodiments said element has the sequence of SEQ ID NO. 1.

In some embodiments, the barcode consists of a payload that can identify the reagent and an error detecting code to validate the correctness of the payload sequence, such as a CRC32 or a Reed-Solomon code.

In embodiments, the reagent according to the present invention is a therapeutic agent selected from the group consisting of tyrosine kinase inhibitors, kinase inhibitors, biologically active agents, biological molecules, radionuclides, adriamycin, ansamycin antibiotics, asparaginase, bleomycin, busulphan, cisplatin, carboplatin, carmustine, capecotabine, chlorambucil, cytarabine, cyclophosphamide, camptothecin, dacarbazine, dactinomycin, daunorubicin, dexrazoxane, docetaxel, doxorubicin, etoposide, epothilones, floxuridine, fludarabine, fluorouracil, gemcitabine, hydroxyurea, idarubicin, ifosfamide, irinotecan, lomustine, mechlorethamine, mercaptopurine, melphalan, methotrexate, rapamycin (sirolimus), mitomycin, mitotane, mitoxantrone, nitrosurea, paclitaxel, pamidronate, pentostatin, plicamycin, procarbazine, rituximab, streptozocin, teniposide, thioguanine, thiotepa, taxanes, vinblastine, vincristine, vinorelbine, taxol, combretastatins, discodermolides, transplatinum, anti-vascular endothelial growth factor compounds ("anti-VEGFs"), antiepidermal growth factor receptor compounds ("anti-EGFRs"), 5-fluorouracil and derivatives, radionuclides, polypeptide toxins, apoptosis inducers, therapy sensitizers, enzyme or active fragment thereof, and combinations thereof.

In some embodiments the reagent according to the present invention is selected from the list of small molecules, peptides, proteins, lipids, polymers, lipid nanoparticles (LNPs), polymers and nucleic acids such as aptamers or combinations thereof.

In some embodiments the candidate library is a DNA-encoded library (DEL).

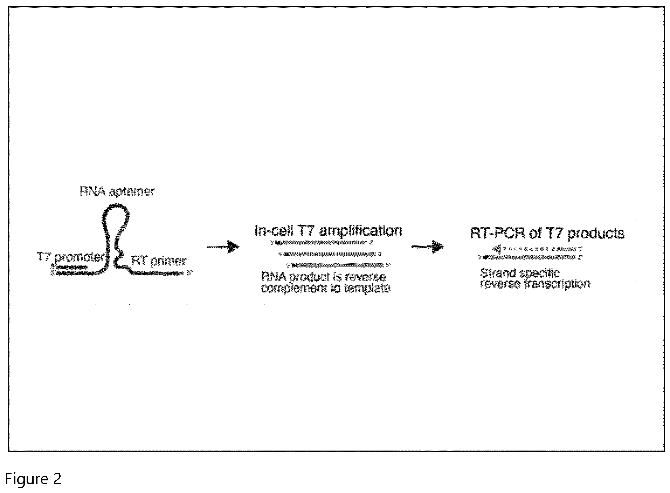

In some embodiments the candidate library is an aptamers library. Aptamer libraries comprise unique nucleic acid sequences that can serve as barcodes and/or amplification initiation elements analogous as the barcodes described according to the present invention. In a preferred embodiments the reagent is an aptamer and in a more preferred embodiment the aptamer simultaneously comprises a barcode and/or amplification initiation element according to the present invention (see also **Figure 2****).** In some embodiments aptamer library size as measured in number of molecules is at least 10 to the power of 1 aptamers, more preferred at least 10 to the power 5 aptamers, even more preferred at least 10 to the power 10 aptamers and most preferred at least 10 to the power 15 aptamers.

In order to stabilize RNA aptamers and improve delivery vehicle *in vivo,* the ribose 2' hydroxyl on the RNA can be replaced with a fluor group, making the RNA unrecognizable by cellular and serum nucleases. In some embodiments RNA aptamers according to the present invention comprises modified nucleotides. In a preferred embodiment, RNA aptamers comprise modifications of the ribose 2' hydroxyl on the RNA backbone selected from the list of 2'OMe nucleotides, 2'-deoxy-2'-fluoro (2'F) nucleotides, 2'-deoxy nucleotides, 2'-O-(2-methoxyethyl) (MOE) nucleotides, locked nucleic acid (LNA) nucleotides, and mixtures thereof. In a more preferred embodiment, RNA aptamers comprise 2'OMe nucleotides, 2'-deoxy-2'-fluoro (2'F) nucleotides modifications, and mixtures thereof.

In a further next step c) target cells from step a) are contacted with the prepared reagent library from step b). Said reagent library is contacted with the target cell in a way that allows interaction of said reagents with the target cells wherein at least a subset of the reagents interacts with at least a subset of the target cells forming cell-reagent complexes. Cell-reagent complexes in this context can be any interaction between reagent and cell such as binding of the reagent to the cell membrane, binding to a cell membrane receptor or active or passive diffusion through the cell membrane.

In some embodiments the ratio of reagents comprising the candidate library to target cells is between 1:10 to the power of 15 and 10 to the power of 15:1.

The reagent library can be contacted with target cells under any condition conductive to form cell-reagent complexes. The condition includes, but is not limited to, for examples, a controlled period of time, an optimal temperature (e.g., 37°C), and/or an incubating medium (e.g., target cell culture medium), etc. In some embodiments the reagent library is contacted with the target cell *in vitro* or *in vivo.* In some embodiments the reagent library is contacted by injection into an organism. In a preferred embodiment the reagent library is contacted with the target cell under physiological conditions to allow binding of aptamers or small molecules to the target cells.

In a subsequent step d) the reagent library is incubated with the target cells to give the reagents the opportunity to interact with the cell surface and to allow entering of the desired subcellular component.

In certain embodiments the candidate reagent library is incubated with the target cells for extended periods of time allowing a subset of reagents to reach desired subcellular compartments in the target cell, thus allowing the degradation of said reagents that show low long-term stability and thereby further selecting and screening for reagents that show high chemical stability within the desired compartment. Non-limiting examples for the period of time of incubation are from about 1 seconds to about 5 days, from about 30 minutes to about 4 days, from about 1 hour to about 3 days, from about 1.5 hours to about 24 hours, or from about 1.5 hours to about 2 hours. In some embodiments, the period of time of incubation may be, for example, 10 min, 15 min, 30 min, 45 min, 1 hour, 2 hours, 4 hours, 6 hours, 8 hours, 12 hours, 16 hours, 24 hours, 2 days, 3 days, 4 days, 5 days, 6 days, 1 week, 1 month, 1 year or 10 years.

In a subsequent step e) the barcodes of reagents that successfully reached the desired cellular compartment are autonomously amplified within said compartment of the host cell by said polymerase expressed in the host cell (in-cell amplification). Said barcodes are amplified only in the cellular compartment to which said polymerase localizes. Consequentially, copy number of barcodes attached to reagents entering the desired compartment are increased with high spatial control. Amplification of barcodes is initiated by the polymerase for instance by recognition of an amplification recognition element sequence comprising the barcode or reagent.

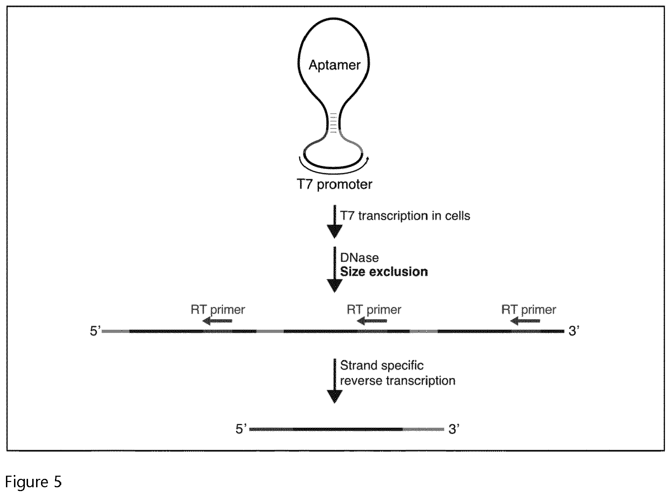

In some embodiments, the amplification according to step e) of the method according to the present invention can lead to either transcription, replication, or rolling circle amplification of barcode sequences generating barcode amplicons with a reverse complement sequence to the original barcodes (see also **Figure 2****,** **5****).**

In step f) of the method according to the invention, following the intracellular amplification of the barcode according to step e), said amplified barcodes are separated from other barcode subsets, thereby identifying and generating a functionally enriched population of barcodes.

In some embodiments, separation is achieved by purifying said amplified barcodes using methods known in the art such as total RNA or DNA extraction from target cells. In some embodiments of the method according to the present invention, a subset of reagents comprising the reagent library that were unable to internalize are separated from the other reagent subsets. In certain embodiments separation is achieved by washing off reagents that did not bind to the target cell surface using washing procedures known in the art. In certain embodiments separation is achieved by washing off reagents that bound target cells but did not internalize using stringent washing procedures known in the art, thereby stripping the cell surface off bound reagents. In a further embodiment the separation is achieved by digesting the barcode of the reagents that did not bind to or interact with the target cell surface using nucleases such as endo- or exonucleases such as DNAses or RNAses.

In some embodiments, the DNA barcodes of reagents such as aptamers that failed to be amplified inside the target cells are degraded using DNA specific nuclease, while the RNA products of in-cell amplification are insensitive to this degradation. In some embodiments, the DNA barcodes contain specific labile bases that can be selectively degraded post in-cell amplification. In some embodiments, the DNA barcodes contain an affinity tag, such as biotin, which facilitates its depletion post in-cell amplification via affinity selection (for example by using Streptavidin coated beads).

In some embodiments the in-cell amplification generates a reverse complement copy of the original barcode. In an embodiment of the method, said barcode further comprises a unique priming site, which is a sequence corresponding to a priming sequence such as a reverse transcription primer (RT primer) site. In some embodiments of the invention, the amplification product is an RNA. This amplification product can serve as the basis for an *in vitro* strand-specific Reverse Transcription PCR (RT-PCR) amplification. In this process a reverse transcription primer, which sequence corresponds to the unique priming site of the barcode amplicon, is provided and enables the priming of the RT reaction. This approach exclusively targets the products of the in-cell amplification (barcode amplicon), and further enhances the copy number of the barcode reverse complement sequence, thereby increasing the signal over the original reagent barcode (see also **Figures 2** to **6****).**

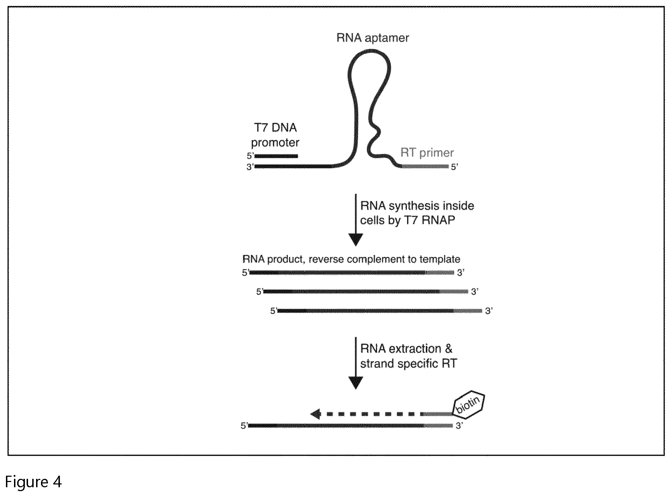

In a preferred embodiment of the method, the RT primer used for RT-PCR is chemically modified and fused to at least one affinity tag. The affinity tag can be any chemical modification such as peptides or proteins as well as small molecules and nucleic acids known in the art for affinity purification of molecules through common methods such as beads or columns chromatography. In a preferred embodiment the RT amplification product is purified using the affinity tag attached to the RT primer (see also **Figure 4****).**

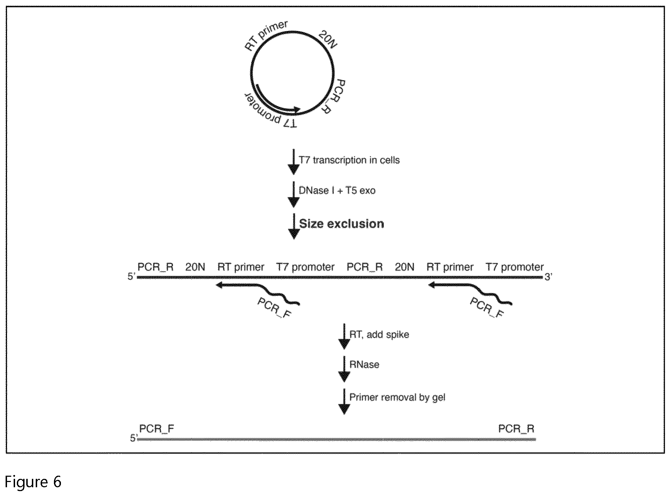

In the case of rolling circle amplification, the in-cell amplification products differ also in size from the original reagent barcode, which can further be used to separate the in-cell amplification progenies of barcodes, via size exclusion chromatography (see also **Figure 5** and **6****).**

In some embodiments, separated barcodes of reagents that successfully penetrated a desired compartment according to step f) can be further amplified and purified prior to identification to further increase sensitivity and exclude undesired barcodes or other nucleic acids contributing to background signal.

In some embodiments, separated barcodes according to step f) are analyzed to identify and/or generate reagents according to the present invention by methods known in the art such as sequencing.

Reagents identified with the method according to the present invention are characterized by the properties of interacting with a target cell and penetrating a desired subcellular compartment. In some cases, it might be useful to further optimize the candidate reagents thus obtained to further identify especially suitable candidate and remove false-positive candidates in an additional screening. In this context, reagents identified by the method according to the present invention can serve as the foundation for a new reagent library. In a further embodiment of the method, said new library can be screened again. In this process the same target cell or any newly selected or generated target cell according to step a) is selected and the process described in steps c) to f) is repeated. In some embodiment, said additional screening is repeated at least once, in a preferred embodiment said additional screening is repeated at least once to 20 times, in a more preferred embodiment said additional screening is repeated at least once to 10 times and in a most preferred embodiment said additional screening is repeated at least once to 5 times.

In some embodiments it is useful to identify candidate reagents that not only localize to a desired compartment but additionally do not localize to one or more different compartments. In an alternative embodiment said reagents are obtainable or identifiable by screening the said newly generated candidate library again in target cells specifically amplifying reagent barcodes of candidates in compartments that are undesirable. Thereby a subset of reagents for said newly generated reagent library can be identified that not only penetrates the desired compartment but also undesired compartments. Said new subset can be subtracted from the initial successful candidate library to further enrich reagents penetrating only the desired compartment.

### INDUSTRIAL APPLICATION

The identified reagents localizing to a desired compartment of a target cell according to the present invention can be used in various applications and methods. Reagents can have intrinsic therapeutical activity and can be used to target subcellular compartments to exert a biological function such as binding target structures e.g., proteins. Reagents identified according to the present invention can be further chemically fused to cargo in order to deliver said cargo to desired compartments of target cells. In this function, the reagent can be designed to target specific cells and/or cellular compartments for cargo delivery by customizing the method in accordance with the present invention. These properties allow the targeted delivery of cargo to many structures previously thought to be undruggable.

Another aspect of the present invention, therefore, refers to a delivery reagent comprising a reagent obtainable by the method according to the present invention and capable of penetrating a desired subcellular compartment of a target cell. In a more preferred embodiment, said reagent is selected from the list of small molecules, peptides, proteins, lipids, polymers, lipid nanoparticles (LNPs), polymers and nucleic acids such as aptamers or combinations thereof. In a preferred embodiment, the present invention relates to a delivery reagent wherein said delivery reagent is an aptamer or a small molecule. In an even more preferred embodiment said reagent is an aptamer.

In a preferred embodiment said delivery reagent is chemically fused to at least one cargo molecule. In a preferred embodiment of the invention cargo is considered any molecule considered for cellular delivery, preferably selected from the list consisting of nucleic acids, proteins, lipids, small molecules, more preferably RNA or DNA, and most preferably siRNAs, gRNAs, IncRNAs, tRNAs, mRNAs, piRNAs and shRNAs.

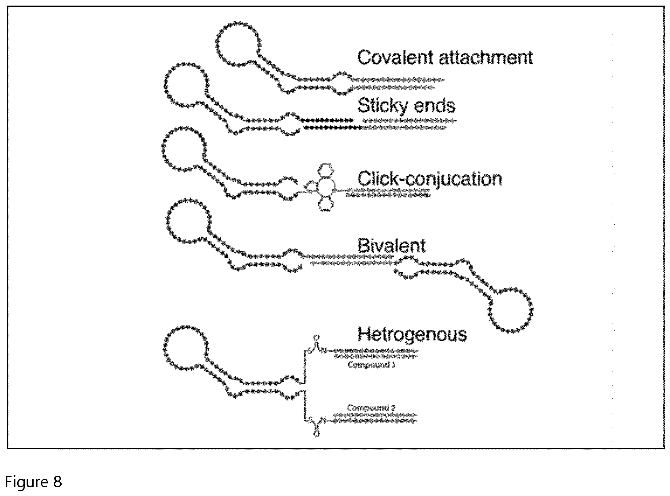

Delivery reagents can be fused to cargo in many ways known in the art. For example, nucleic acid cargo can be covalently attached to the reagent by click chemistry. Additionally, multiple identical or different molecules of cargo can be attached to the delivery reagent. For instance, but not limiting, two molecules of heterologous cargo can be attached to a delivery reagent such as an aptamer obtainable by the method according to the present invention (see also **Figure 8****).**

In some embodiments, multiple reagents obtainable by the method of the present invention can be chemically attached. For instance, multiple aptamers can be bivalently or multivalently conjugated (see also **Figure 8****).**

Another object of the present invention refers to a method to deliver cargo to target cells comprising the following steps:
a) providing a target cell in need of cargo delivery;
b) providing the delivery reagent according to the invention;
c) chemically attach said cargo to said reagent and
d) contacting said target cells with the reagent thus obtained.

The delivery reagent according to the present invention can be used as transfection reagent able to deliver cargo such as nucleic acids to selected cells.

The delivery reagent according to the present invention can be further used as a medicament or therapeutical. For instance, the reagent can be fused or conjugated to therapeutic drugs or other effector molecules.

The concept of cargo delivery by biological reagents such as antibodies specifically targeting structures on target cells is well known. In this process, antibodies are conjugated to effector molecules forming so called immunoconjugates, which have been intensively used as therapeutics to treat medical conditions such as cancer.

Aptamers show functionally similar properties to antibodies. However, it is widely accepted that their absorption, distribution, metabolism, and excretion ("ADME") properties are intrinsically different. Most notably, aptamers show much lower immunogenic properties in comparison to antibodies, which can for instance induce antibody-dependent cellular cytotoxicity or complement-dependent cytotoxicity.

In comparing many of the properties of aptamers and antibodies previously described, several factors suggest that cargo-delivery via aptamers offers several concrete advantages over delivery with antibodies, ultimately affording them better potential as therapeutics. Several examples of the advantages of cargo-delivery via aptamers over antibodies are as follows:
1. Aptamer-cargo conjugates are entirely chemically synthesized. Chemical synthesis provides more control over the nature of the conjugate. For example, the stoichiometry (ratio of cargo per aptamer) and site of attachment can be precisely defined. Different linker chemistries can be readily tested. The reversibility of aptamer folding means that loss of activity during conjugation is unlikely and provides more flexibility in adjusting conjugation conditions to maximize yields.
2. Smaller size allows better tissue penetration. Poor penetration of antibodies into solid tumors for instance is often cited as a factor limiting the efficacy of conjugate approaches (Colcher, D., Goel, A., Pavlinkova, G., Beresford, G., Booth, B., Batra, S. K. (1999) "Effects of genetic engineering on the pharmacokinetics of antibodies", Q. J Nucl. Med., 43: 132-139). Studies comparing the properties of unPEGylated anti-tenascin C aptamers with corresponding antibodies demonstrate efficient uptake into tumors (as defined by the tumor:blood ratio) and evidence that aptamer localized to the tumor is unexpectedly longlived (t1/2>12 hours) (Hicke, B. J., Stephens, A. W., "Escort aptamers: a delivery service for diagnosis and therapy", J. Clin. Invest., 106:923-928 (2000)).
3. Tunable PK. Aptamer half-life/metabolism can be easily tuned to match properties of payload, optimizing the ability to deliver toxin to a tissue while minimizing systemic exposure. Appropriate modifications to the aptamer backbone and addition of high molecular weight PEGs should make it possible to match the half-life of the aptamer to the intrinsic half-life of the conjugated toxin/linker, minimizing systemic exposure to nonfunctional toxin-bearing metabolites (expected if t1/2(aptamer)<<t1/2(toxin)) and reducing the likelihood that persisting unconjugated aptamer will functionally block uptake of conjugated aptamer (expected if t1/2(aptamer)> >t1/2(toxin)).
4. Relatively low material requirements. It is likely that dosing levels will be limited by toxicity intrinsic to the cytotoxic payload. As such, a single course of treatment will likely entail relatively small (< 100 mg) quantities of aptamer, reducing the likelihood that the cost of oligonucleotide synthesis will be a barrier for aptamer-based therapies.

In some embodiments the present invention relates to a delivery reagent comprising a reagent obtainable by the method according to the invention and capable of penetrating a desired subcellular compartment of a target cell. In a further embodiment the present invention relates to a delivery reagent comprising a nucleic acid capable of binding a target cell, inducing endocytosis and internalization into said target cell and subsequent endosomal escape, wherein the nucleic acid is an aptamer obtainable according to the method of the present invention.

It has been surprisingly found that reagents according to the present invention are able to deliver cargo to target cells and, in contrast to immunoconjugates, show high release rates to cellular compartments such as the cytoplasm allowing the possibility to target structures previously out of reach.

The present disclosure provides a pharmaceutical composition comprising a therapeutically effective amount of a delivery reagent provided by the invention, optionally attached to at least one cargo molecule, or a salt thereof, and a pharmaceutically acceptable carrier or diluent. Relatedly, the present disclosure provides a method of treating or ameliorating a disease or disorder, comprising administering the pharmaceutical composition to a subject in need thereof.

Administering a therapeutically effective amount of the composition to the subject may result in: (a) an enhancement of the delivery of cargo to a disease site or a subcellular compartment of a target cell relative to delivery of the cargo alone; or (b) an enhancement of target clearance resulting in a decrease of at least 10%, 20%, 30%, 40%, 50%, 60%, 70%, 80%, or 90% in a blood level of the target of the reagent, e.g., a protein; or (c) an decrease in biological activity of the target of the reagent, e.g., a protein, of at least 10%, 20%, 30%, 40%, 50%, 60%, 70%, 80%, or 90%.

Administering a therapeutically effective amount of the pharmaceutical composition according to the invention can be achieved by any means known such as intravenously, subcutaneously, intraperitoneally, orally, or through inhalation.

The disease or disorder can include without limitation those disclosed herein. The disease or disorder may include without limitation COVID19, Influenza, Breast Cancer, Alzheimer's disease, bronchial asthma, Transitional cell carcinoma of the bladder, Giant cellular osteoblastoclastoma, Brain Tumor, Colorectal adenocarcinoma, Chronic obstructive pulmonary disease (COPD), Squamous cell carcinoma of the cervix, acute myocardial infarction (AMI) / acute heart failure, Chron's Disease, diabetes mellitus type II, Esophageal carcinoma, Squamous cell carcinoma of the larynx, Acute and chronic leukemia of the bone marrow, Lung carcinoma, Malignant lymphoma, Multiple Sclerosis, Ovarian carcinoma, Parkinson disease, Prostate adenocarcinoma, psoriasis, Rheumatoid Arthritis, Renal cell carcinoma, Squamous cell carcinoma of skin, Adenocarcinoma of the stomach, carcinoma of the thyroid gland, Testicular cancer, ulcerative colitis, or Uterine adenocarcinoma.

Delivery reagents such as aptamers according to the present invention are particularly useful for treatment of pulmonary diseases caused by infection. Aptamers can be modified to increase chemical stability and functionally conjugated to cargo such as siRNAs.

A further embodiment, therefore, refers to a delivery reagent obtainable according to the present invention as a medicament or for use in therapy of pulmonary disease. In particular, aptamers obtainable according to the present invention can be selected for selective delivery of therapeutics to for instance the lung epithelia, which is commonly targeted by many pathogens such as viruses e.g., SARS-CoV-2. The present invention therefore discloses pharmaceutical compositions useful to treat pulmonary diseases. In a preferred embodiment, the pharmaceutical embodiment comprises an aptamer fused to therapeutical RNA such as a siRNA useful to treat COVID19.

The invention will be explained in more detail by virtue of the examples set forth herein below. While at least one exemplary embodiment is presented, it should be appreciated that a vast number of variations exist. It should also be appreciated that the exemplary embodiments are only examples, and are not intended to limit the scope, applicability, or configuration in any way. Rather, the forgoing description will provide those with ordinary skill in the art with the essential characteristics of this invention for implementing at least one exemplary embodiment, it being understood that various changes may be made without departing from the scope as set forth in the appended claims.

### BRIEF DESCRIPTION OF THE FIGURES

**Figure 1** shows a schematic representation of a possible embodiment of the method. Target cells expressing the polymerase T7 transgene are challenged with a reagent library of 10 to the power of 15 aptamers. Aptamers that enter the cell and escape to the cytoplasm are amplified by the T7 polymerase, improving the signal to noise ratio. The cells are then thoroughly washed to remove the excess of non-specific aptamers. Finally, the extracted RNA undergoes a selective RT-PCR that targets only the negative strand T7 amplification products.
**Figure 2** shows a further aspect of an embodiment of the method, an example for the design of a possible reagent, said reagent being an aptamer. The aptamer serves as reagent as well as barcode in this example. The aptamer has a T7 promoter [left] and an RT primer binding site [right]. Cell-penetrating aptamers are amplified, creating a reverse complement copy [middle]. The RT-PCR targets RT primer binding site on the reverse complement strand of the aptamer for strand-specific amplification. Amplified sequences can be further separated and analyzed by for instance sequencing.

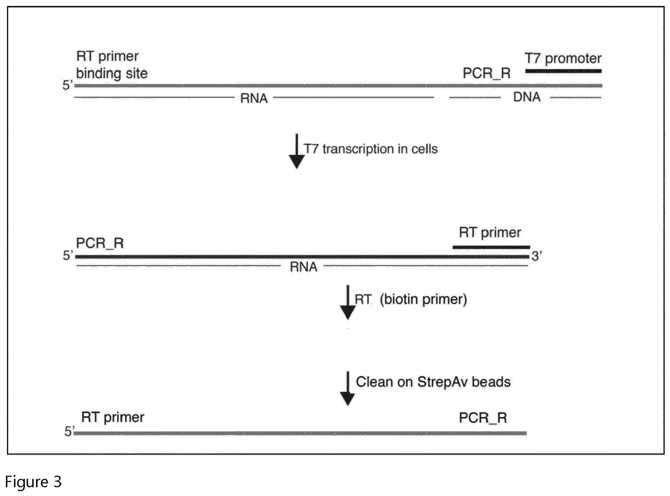

**Figure 3** illustrates an embodiment of the present invention and exemplarily describes a possible separation strategy of amplified barcodes according to step f). In this example, the barcode consists of a dshoDNA (double stranded homoduplex DNA) /ssRNA chimera aptamer, wherein the DNA part comprises a T7 promoter and the RNA part further comprises a RT primer binding site. The barcode sequence is represented by the overall sequence of the aptamer, which in this context represents the reagent. Following in-cell amplification according to step e) using a T7 polymerase expressed ubiquitously and localizing to the cytoplasm, the amplicons are RNA molecules with a reverse complement sequence in regards of the original barcode. Said amplicons can be selectively reverse transcribed yielding a single strand DNA molecule, which can be optionally further purified before analyzing the sequence of the aptamer in order to identify said reagent according to the present invention, wherein said reagents represents an aptamer able to bind, internalize and escape from the endosome of a target cell.
**Figure 4** illustrates a possible embodiment of the present invention in which the reagent according to the present invention relates to an aptamer analogous to Figure 3. Said aptamer being in-cell amplified by T7 RNA polymerase (T7 RNAP). The amplicon is further reverse transcribed using a RT primer binding the RT binding site contained within the aptamer sequence. Said RT primer further comprising an affinity tag such as biotin. Reverse transcribed DNA molecules can be further purified using methods known in the art such as streptavidin-affinity-chromatography in order to purify said barcodes and increase signal to noise ratio in subsequent analysis methods such as sequencing, by removing remnants of non in-cell amplified aptamers.
**Figure 5** shows a further embodiment of the present invention. The reagent according to the invention can be a circular aptamer, comprising an amplification initiation site such as a T7 promoter. In-cell amplification according to step e) can be rolling-circle amplification resulting in long stretches of repetitive sequences in reverse complement orientation to the aptamer sequence. Said repetitive sequences can be RNA molecules. DNA aptamers that failed to be amplified can be digested using DNase. Large fragments can be separated from low molecular weight aptamers that failed to amplify and further selectively reverse transcribed. Reverse transcribed DNA fragments can be analyzed according to methods known in the art such as sequencing.
**Figure 6** shows an example for a circular DNA aptamer according to the present invention comprising a barcode sequence, an amplification initiation element, a T7 promoter, which is recognized by a T7 RNAP according to the invention. In-cell amplification results in the generation of repetitive RNA stretches in reverse complement orientation in regards to the original aptamer sequence. The reverse complement amplicons reveal an accessible RT primer site, which is bound by RT primers in order to initiate reverse transcription. Following reverse transcription, generated DNA fragments can be isolated and analyzed according to methods known in the art such as sequencing.

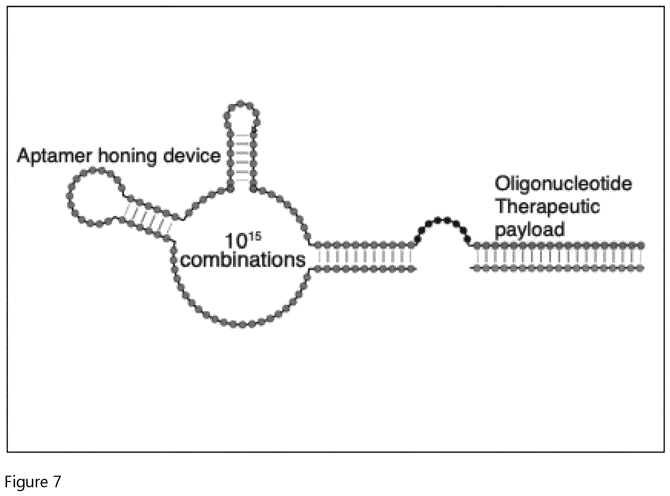

**Figure 7** shows a further embodiment of the present invention. Shown is an example for a delivery reagent obtainable according to the method of the present invention further conjugated to cargo. The reagent can be comprised of an aptamer identified from a reagent library according to the method. Said reagent can be conjugated to cargo such as oligonucleotides (e.g., siRNAs). The delivery reagent fused to cargo can be useful as a therapeutic reagent delivering cargo to a desired subcellular compartment of a target cell by means of the reagent identified according to the method of the present invention.
**Figure 8** illustrates further embodiments of the present invention. Delivery reagents identified according to the present invention such as aptamers can be fused to cargo such as nucleic acids in many ways known in the art. For example, nucleic acid cargo can be covalently attached to the aptamer, bind through sticky ends, conjugated by click chemistry. Furthermore, multiple aptamers can be bivalently or multivalently conjugated. Additionally, multiple identical or different molecules of cargo can be attached to the delivery reagent. For instance, but not limiting, two molecules of heterologous cargo can be attached to a delivery reagent such as an aptamer obtainable by the method according to the present invention.

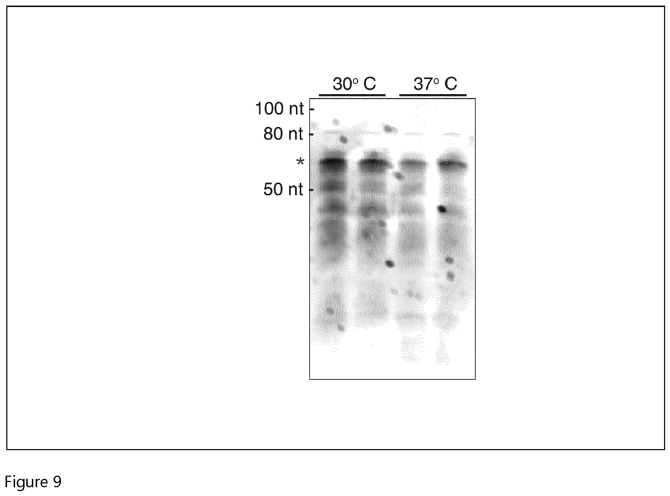

**Figure 9** illustrates the result obtained for testing the ability of T7 RNA polymerase (RNAP) to utilize a single stranded RNA aptamer covalently linked to a double stranded DNA T7 promoter as a template for *in vitro* transcription. Shown is a TBE-Urea gel with sizes of nucleic acid fragments shown for *in vitro* transcription at 30°C and 37°C respectively.

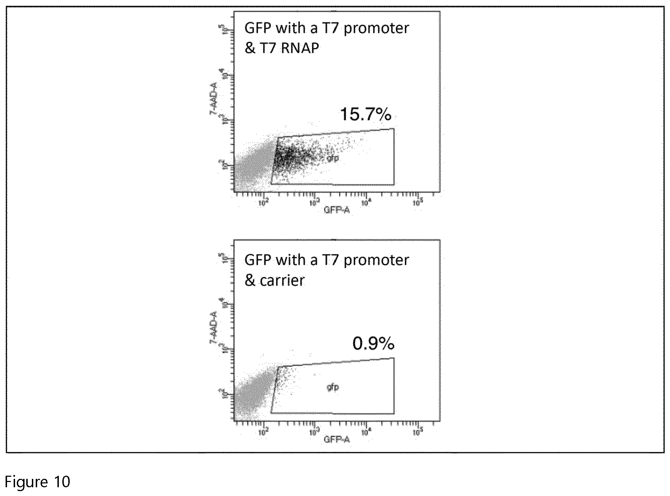

**Figure 10** shows cell cytometry results of cells ectopically expressing T7 polymerase with a GFP-reporter gene under the control of the T7 amplification sequence (T7 promoter). Additionally, a negative control, not expressing T7 polymerase was included (lower panel). GFP positive cell populations were gated and shown is the percentage of cells expressing GFP.

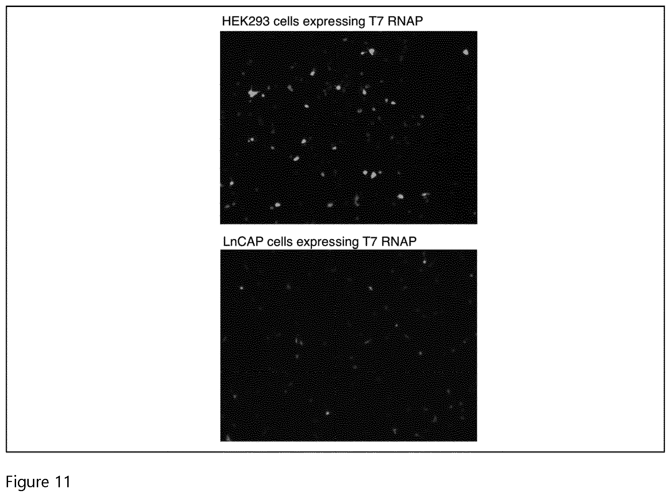

**Figure 11** shows fluorescence microscopy images of two cell lines expressing T7 RNA polymerase (T7 RNAP). Signal is shown in white.

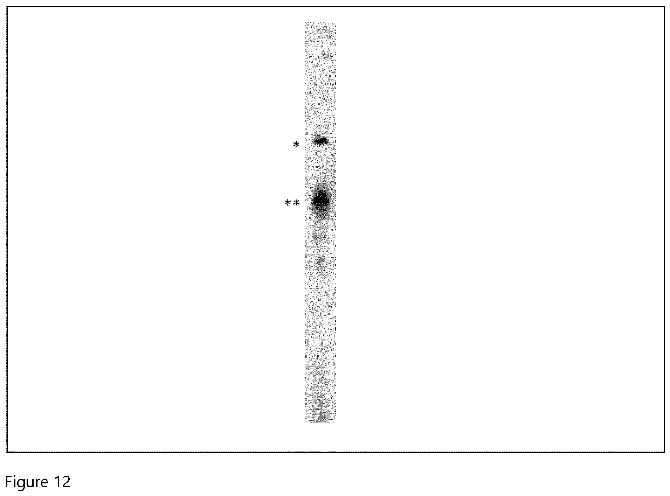

**Figure 12** shows the results obtained for testing the ability of T7 RNA polymerase (RNAP) to utilize a single stranded 2'fluoro modified RNA aptamer as a template for highly effective transcription *in vitro.* Shown is a TBE-Urea gel, one star denotes the expected size of the 2'fluoro RNA-DNA template. Two-stars denotes the expected size of the resulting RNA product (of note, the T7 amplification product doesn't include the promoter and is therefore shorter).

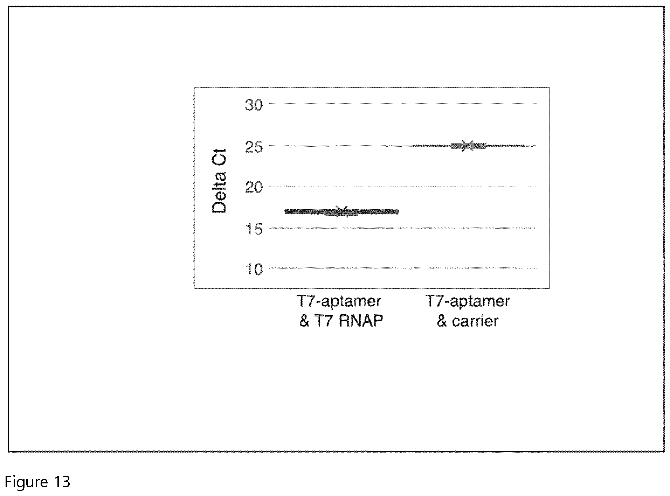

**Figure 13** shows the results of a qPCR experiment indicating the differences in delta Ct values and cycle number of in-cell amplification by cells either expressing a T7-controlled barcode sequence (in this case an RNA aptamer) and T7 polymerase or a negative control expressing the T7-controlled RNA aptamer only. The data presented is from three biological replicates. Indicated are the delta Ct values of the qPCR experiment, normalized to a housekeeping gene (18S ribosomal RNA).

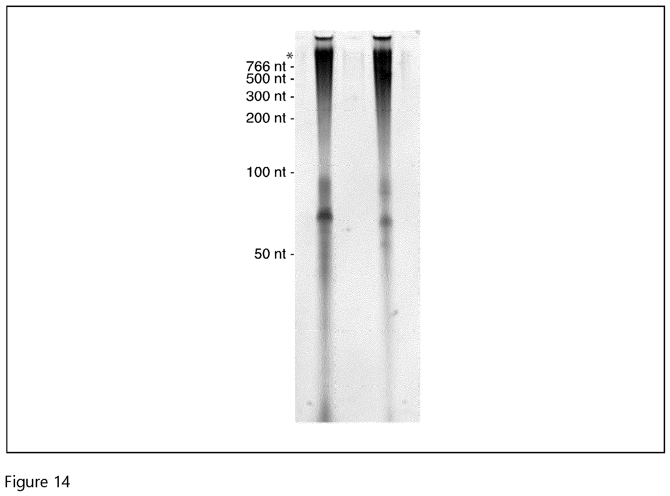

**Figure 14** shows results obtained for testing the ability of T7 RNA polymerase (RNAP) to utilize a circular single stranded RNA aptamer as a template for *in vitro* transcription. Shown is a TBE-Urea gel with sizes of nucleic acid fragments shown for *in vitro* transcription. Star denotes high molecular weight concatemeric RNA products resulting from rolling circle amplification by T7.

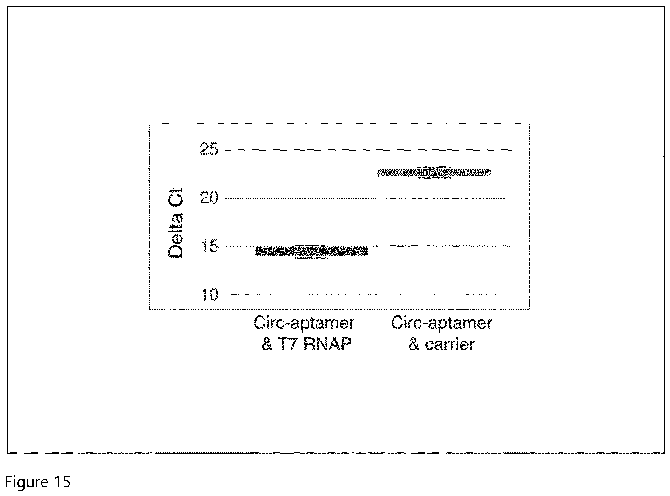

**Figure 15** illustrates the results of a qPCR experiment indicating the differences in delta Ct values of in-cell amplification by cells either expressing a T7-controlled circular barcode sequence (in this case aptamer) and T7 polymerase or a negative control expressing the T7-controlled circular barcode only. Shown is the quantification for three biological replicates.

### EXAMPLES

### Example 1

### T7 In vitro transcription of RNA aptamers

The improvement of sensitivity for the identification of reagents that are able to localize to a desired compartment within a target cell according to the present invention relies in part on the efficient amplification of barcodes attached to said reagents within the cell. Barcodes such as aptamers are necessary to identify the reagents according to the present invention. In order to test the efficiency of amplification of said barcodes, the ability of T7 RNA polymerase (RNAP) to utilize a single stranded RNA aptamer as a template for *in vitro* transcription was assessed.

To do so a 42 nucleotide long single stranded RNA fused on its 3' to a 36 nt long DNA was used as a template for *in vitro* T7 transcription. The 5' end of the DNA was single stranded while the 3' end of the DNA was a double stranded T7 promoter (see also Figure 2). *In vitro* transcription was carried out by HiScribe^{™} T7 High Yield RNA Synthesis Kit (NEB) according to the manufacturer recommendations, in either 30 or 37 Celsius degrees, after which the resulting RNA was assessed for quantity by Qubit^{™} RNA BR Assay Kit (Invitrogen) and visualized on a Novex^{™} TBE-Urea Gels, 15% (Invitrogen).

The data presented in Figure 9 show that T7 RNAP is capable of utilizing a single stranded RNA aptamer as a template for highly effective transcription *in vitro.* Star denotes the expected size of the resulting RNA product.

### Example 2

### In-cell amplification of RNA aptamer

While aptamers according to the present invention were able to be transcribed in *vitro,* the method according to the present invention requires amplification within the target cell and optionally in a desired subcellular compartment. Therefore, the ability of in-cell amplification of barcodes such as aptamers was assessed.

HEK293 cells were co-transfected with (i) a T7 RNAP mammalian expression plasmid, along with (ii) a plasmid encoding a GFP reporter gene under the control of a T7 promoter, or a carrier plasmid that do not encode for T7 RNAP as a control. All transfections were performed using Lipofectamine^{™} 3000 Transfection Reagent (Invitrogen) according to the manufacturer recommendations. GFP expression was assessed 30 hours following transfection by Cell Cytometry (Figure 10) or fluorescence microscopy (Figure 11). Fluorescence microscopy was additionally performed for a second cell line (LnCAP) expressing a T7 RNAP expression plasmid, along with a plasmid encoding a GFP reporter gene under the control of a T7 promoter.

The data presented in Figure 10 and 11 show that T7 RNAP ectopically expressed in human cells is catalytically active and can transcribe RNA from a T7 promoter inside cells. These results illustrate that in-cell amplification of a barcode such as aptamer according to the present invention is highly efficient and suitable to increase copy numbers of barcodes thereby increasing signal to noise ratio over background.

### Example 3

### In-cell amplification and separation of RNA aptamers

Following successful in-cell amplification of barcodes such as aptamers in the desired subcellular compartment of target cells according to the present invention, amplification products are separated. This can be achieved by isolation of nucleic acids such as DNA and/or RNA from target cells. In some embodiments of the method, the amplification products are reverse complement RNA transcripts of barcodes successfully localizing to the desired compartment of the target cell specifically generated by the polymerase according to the invention. As described (see also Figure 3), the reverse complement transcript of the barcode can comprise a RT primer binding site, which binds a RT primer able to selectively reverse-transcribe the RNA amplicon product into DNA. Said DNA can then be further separated from the reaction and analyzed by quantitative and qualitative means. In some embodiments the RT primers are attached to biotin molecules for streptavidin-mediated cleanup of the RT products that comprise barcode amplicons and remove remnants of aptamers that failed to be amplified inside the target cells. Quantification can be achieved by qPCR, thereby evaluating the successful in-cell amplification and subsequent separation procedure according to the present invention. The objective of the example described below was to test the ability of in-cell amplification and efficient separation of barcodes, such as aptamers, in living cells.

In a first step, the DNA-RNA aptamer T7 chimera shown in Figure 2 was co-transfected into HEK293 cells along with a plasmid encoding for T7 RNAP, or with a control plasmid, using Lipofectamine^{™} 3000 Transfection Reagent (Invitrogen) according to the manufacturer recommendations. Following 30 hours incubation, cellular RNA was extracted using a Quick-RNA Miniprep Kit (Zymo Research). DNA remnants were digested using TURBO^{™} DNase (Invitrogen). In-cell T7 amplification products were reverse transcribed using a strand specific biotinylated primer and superscript^{™} III Reverse Transcriptase (Invitrogen). A second, biotinylated reverse transcription primer targeting the 18S ribosomal RNA was also added to the same reactions - to serve as a housekeeping normalization control. To remove cDNA produced by random RNA priming, the resulting cDNA was purified using Dynabeads^{™} MyOne^{™} Streptavidin C1 beads, and was used as a template for quantitative PCR reactions targeting the in-cell T7 amplification products of the aptamers, or the 18S ribosomal RNA control. Average qPCR delta Ct values [aptamer cycle threshold - 18S rRNA cycle threshold] of three biological replicates were recorded.

The data is presented in Figure 12 and 13 and demonstrate effective in-cells T7 amplification of an RNA aptamer, and selective *in vitro* amplification of the in-cell T7 products, with 8 PCR cycles difference from control cells without T7 RNAP. This shows the ability to select and further amplify aptamers that reached the desired subcellular compartment such as the cell cytoplasm and become available to interact with cytoplasmic proteins.

### Example 4

### T7 In vitro transcription of circular DNA aptamer

In some embodiments of the present invention, the barcode or aptamer can be a circular nucleic acid such as a single stranded circular DNA sequence. The objective of this example was to test the ability of T7 RNAP to utilize a single strand DNA circle as a template for rolling circle amplification *in vitro* according to the present invention.

A 70 nucleotide long single stranded DNA circle annealed to aT7 promoter (as shown in Figure 6) was used as a template for *in vitro* T7 transcription by HiScribe^{™} T7 High Yield RNA Synthesis Kit (NEB) according to the manufacturer recommendations. Following removal of the DNA template by TURBO^{™} DNase (Invitrogen), the resulting RNA was visualized on a Novex^{™} TBE-Urea Gels, 15% (Invitrogen).

The results are presented in Figure 14. The data show that T7 RNAP is capable of utilizing a single strand DNA circle as a template for rolling circle amplification *in vitro.* Star denotes high molecular weight concatemeric RNA products resulting from rolling circle *in vitro* amplification by T7.

### Example 5

### T7 In vitro transcription of 2'fluoro modified RNA aptamer

In order to stabilize RNA aptamers and make them a better delivery vehicle *in vivo,* the ribose 2' hydroxyl on the RNA can be replaced with a fluor group (2'F), making the RNA unrecognizable by cellular and serum nucleases. In order to test the ability of amplification of said barcodes made of 2'fluoro modified RNA, the ability of T7 RNA polymerase (RNAP) to utilize a single stranded 2'fluoro modified RNA aptamer as a template for *in vitro* transcription was assessed.

To do so, a 42 nucleotide long single stranded RNA fully modified with 2'fluoro in all positions was fused on its 3' to a 36 nt long DNA and was used as a template for *in vitro* T7 transcription. The 5' end of the DNA was single stranded while the 3' end of the DNA was a double stranded T7 promoter (see also Figure 2). *In vitro* transcription was carried out by HiScribe^{™} T7 High Yield RNA Synthesis Kit (NEB) according to the manufacturer recommendations in either 37 Celsius degrees, after which the resulting RNA was assessed for quantity by Qubit^{™} RNA BR Assay Kit (Invitrogen) and visualized on a Novex^{™} TBE-Urea Gels, 15% (Invitrogen).

The data presented in Figure 12 show that T7 RNAP is capable of utilizing a single stranded 2'fluoro modified RNA aptamer as a template for highly effective transcription *in vitro.* Star denotes the expected size of the 2'fluoro RNA-DNA template. Two-stars denotes the expected size of the resulting RNA product (of note, the T7 amplification product doesn't include the promoter and is therefore shorter).

### Example 6

### In-cell amplification of circular DNA

Following the demonstration that T7 polymerase is able to amplify circular barcodes resulting in concatemeric RNA products, in this example the ability of in-cell amplification using said circular barcodes was evaluated. To do so, in cell T7 rolling circle amplification of circular single stranded DNA aptamers, and strand-selective *in vitro* amplification of the T7 products was performed. Transcription of circular DNA can result in repetitive transcripts of the target nucleic acid forming long stretches. Said stretches can be size selected by gel chromatography thereby purifying amplification products for further analysis.

The single stranded DNA circular aptamer shown in Figure 6 was co-transfected into HEK293 cells along with a plasmid encoding for T7 RNAP, or with a control plasmid, using Lipofectamine^{™} 3000 Transfection Reagent (Invitrogen) according to the manufacturer recommendations. Following 30 hours incubation, cellular RNA was extracted using a Quick-RNA Miniprep Kit (Zymo Research). DNA remnants were digested using TURBO^{™} DNase (Invitrogen). In order to further eliminate remnants of the original circular aptamer library, the extracted RNA was loaded on a Novex^{™} TBE-Urea Gels, 10% PAGE (Invitrogen), and RNA larger than 350 nt long was excised for further analysis. In cell T7 products were reverse transcribed using a strand specific primer and superscript^{™} III Reverse Transcriptase (Invitrogen). The resulting cDNA was used as a template for quantitative PCR reactions targeting the in cell T7 amplification products of the aptamers.

The results of the qPCR are shown in Figure 15. Average qPCR cycle thresholds of three biological replicates are shown. The data demonstrate effective T7 rolling circle amplification of a single stranded circular aptamer inside human cells, and selective *in vitro* amplification of the in-cell T7 products, with 8 PCR cycles difference from control cells without T7 RNAP. This shows the ability to select and further amplify aptamers that reached the cell cytoplasm and become available to interact with cytoplasmic proteins.

## Claims

1. A method for generating and/or identifying a reagent, said reagent being able to enter a desired subcellular compartment of a target cell, comprising the following steps:
a. preparing and/or selecting target cells comprising a polymerase localizing to said desired compartment;
b. preparing a candidate library of reagents comprising or consisting of nucleic acid barcodes recognized by said polymerase;
c. contacting said target cells with said library of reagents, wherein at least a subset of reagents interacts with at least a subset of target cells forming cell-reagent complexes;
d. incubating the cell-reagent complexes thus obtained for a period of time at least sufficient to allow at least a subset of said reagents to enter a desired subcellular compartment of a target cell;
e. amplifying the nucleic acid barcode of said subset of reagents of step d) by said polymerase of step a) within the desired subcellular compartment of the target cells and
f. separating the amplified nucleic acid barcodes of step e) to generate and/or identify said reagent.

2. The method according to claim 1, wherein said candidate library of reagents is a DNA-encoded library.

3. The method according to claim 1, wherein said candidate library of reagents is an aptamer library.

4. The method according to any of claims 1 to 3, wherein said polymerase is a T7 RNA polymerase.

5. The method according to any of claims 1 to 4, wherein said barcode comprises at least one amplification initialization element.

6. The method according to claim 5, wherein said amplification initialization element is a T7 promoter.

7. The method according to claim 6, wherein said T7 promoter has the sequence of SEQ ID NO. 1.

8. The method according to any of claims 1 to 7, wherein the barcode further comprises a reverse transcription primer site.

9. The method according to any one of claims 1 to 8, further comprising step g):
i. preparing a new candidate library of reagents from the identified reagents of step f); and
ii. repeating steps a) and c) to f) using said newly prepared candidate library of reagents,
wherein step g) is repeated at least 1 time.

10. The method according to any one of claims 1 to 9 further comprising identifying the reagent of step f) thus obtained by sequencing.

11. A delivery reagent comprising a reagent obtainable by the method according to any of claims 1 to 10 and capable of penetrating a desired subcellular compartment of a target cell.

12. A delivery reagent according to claim 11, wherein said delivery reagent is an aptamer or a small molecule.

13. A delivery reagent according to any claims 11 to 12, wherein the reagent is fused to at least one cargo molecule.

14. A pharmaceutical composition comprising a therapeutically effective amount of a delivery reagent according to any of claims 11 to 13.

15. The use of the delivery reagent of any one of claims 11 to 13 as a medicament or for use in therapy of pulmonary disease.
